# EUROPÄISCHE PATENTSCHRIFT

(11) **EP 0 856 312 B1**
(45) Veröffentlichungstag und Bekanntmachung des Hinweises auf die Patenterteilung: **14.10.1998**
(21) Anmeldenummer: 96118939.6
(22) Anmeldetag: 27.11.1996
(51) Int. Cl.: A61K 31/46

(54) **Verwendung von Trospiumchlorid zur Herstellung eines Arzneimittels zur Behandlung von Blasenkrankheiten**
Use of trospium chloride for the manufacture of a medicament for the treatment of diseases of the bladder
Utilisation du chlorure de trospium pour la fabrication d'un médicament pour le traitement de maladies de la vessie

(43) Veröffentlichungstag der Anmeldung: 05.08.1998
(73) Patentinhaber: Dr. R. Pfleger Chemische Fabrik GmbH, 96052 Bamberg (DE)
(72) Erfinder: Schwantes. Ulrich, Dr.rer.nat., D-96129 Geisfeld (DE); Schaupp. Albert, D-96129 Amlingstadt (DE); Stöhrer, Manfred, Dr.med.habil., D-82418 Murnau (DE)
(74) Vertreter: Fleck, Thomas, Dr. Dipl.-Chem.

(56) Entgegenhaltungen:
- DE-A- 3 546 165
- "Intravesikale Spasmolytika" UROLOGE AUSG. B, Bd. 34, Nr. 2, 1994, Seite 95 XP002025014
- INGRID E. NYGAARD: "Nonoperative management of urinary incontinence" CURRENT OPINION IN OBSTETRICS AND GYNECOLOGY, Bd. 8, Nr. 5, 1996, Seiten 347-350, XP000650525
- F.MATZKIES ET AL.: "Ultrasound studies of the effect of trospium chloride on gall-bladder kinetics" ARZNEIMITTELFORSCHUNG, Bd. 42, Nr. 12, 1992, Seiten 1456-1458, XP002025015
- Urologie in Klinik und Praxis, 1982, Seiten 290-325
- J.Urol., Band 148, 1992, S. 595-597
- Eur. Urol., Band 28, 1995, S. 340-344
- J.Urol., Band 155, 1996, S. 768-771
- J.Urol., Band 157, 1997, S. 638-640

## Beschreibung

Die vorliegende Erfindung betrifft die Verwendung von Trospiumchlorid zur Herstellung einer Arzneimittelzubereitung auf Basis einer sterilen, wässrigen Lösung bei der Behandlung von Blasenfunktionsstörungen und des Harnsystems, sowie ein 2-Komponenten-System hierfür.

Der Wirkstoff Trospiumchlorid ist als Anticholinergikum (Spasmolytikum) seit mehreren Jahrzehnten bekannt (vgl. hierzu DE-PS 1 194 422). Dieser Wirkstoff wurde als oral zu applizierende feste Darreichungsform (Tabletten und Dragées), zur intravenösen, bzw. intramuskulären Injektion als Injektionslösung und zur rektalen Applikation als Suppositorien angeboten und in erster Linie zur Behandlung von Harnblasenfunktionsstörungen (Urge-Inkontinenz, Detrusor-Hyperreflexie) angewendet. Bei diesen Applikationsformen traten wärend des Wirkstofftransportes vom Applikationsort zum Wirkort, bedingt durch die während der systemischen Passage ablaufenden Prozesse der Exkretion und des Metabolismus, bei den oralen und rektalen Darreichungsformen zudem bedingt durch die Absorptionsverluste dieser quartären Ammoniumverbindung bei der Aufnahme des Wirkstoffes aus dem Darmlumen in das System, Wirkstoffverluste auf. Außerdem haben sich bei diesen Arten der Wirkstoffapplikation die für Anticholinergika typischen Nebenwirkungen, wie Steigerungen der Herzfrequenz, Mundtrockenheit, Akkomodationsstörungen usw. nachteilig bemerkbar gemacht.

Andere Anticholinergika, die von ihrer Molekularstruktur tertiäre Amine darstellen (z.B. Oxybutynin), werden nach intravesikaler Instillation in größerem Ausmaß absorbiert als nach Applikation equivalenter oraler Dosierungen (Massad C.A., Kogan B.A. & Trigo-Rocha F.E.: The Pharmacokinetics of intravesical and oral oxybutynin chloride J. Urol. 148: 595-597, 1992). Bei diesen Substanzen stellt die intravesikale Instillation des Wirkstoffes somit keinen geeigneten Weg zur Limitierung der unerwünschten Nebenwirkungen dar, da diese hinsichtlich ihres Auftretens und hinsichtlich ihrer Intensität in erster Linie abhängig von den systemischen Wirkspiegeln am Zielorgan (.B. Herz, Speicheldrüse, Auge) sind.

Urologe [B], 1994, Band 34, S. 95 empfiehlt die lokale Instillation von Spasmex® in die Blas.

Der vorliegenden Erfindung liegt deshalb die Aufgabe zugrunde, die Nachteile der bisherigen Trospium-Applikationen auszuschließen und dennoch die Applikation durch den Patienten selbst beizubehalten, so daß auf klinisches Personal oder gar einen Arzt selbst verzichtet werden kann.

Diese Aufgabe wird durch die erfindungsgemäße Verwendung gemäß Anspruch 1 oder das erfindungsgemäße 2-Komponenten-System nach Anspruch 2 gelöst, die ausgezeichnete Ergebnisse liefern und die Nachteile der vorbekannten Applikationsformen ausschließen. Damit die einzusetzende wässrige Lösung über längere Zeit während der Lagerung und des Transportes stabil bleibt, müßte der pH-Wert eigentlich kleiner als 4,5 betragen. Eine solche Lösung ist jedoch niemals zur Applikation in die Blase hinein als geeignet angesehen worden und auch aus physiologischen Gründen nicht akzeptabel. Die Erfindung geht deshalb davon aus, daß die wässrige Lösung mit einem pH-Wert größer oder = 4,5 vor der Applikation zubereitet wird, um Alterungs-, Lagerungs- und Transportprobleme von vornherein auszuschließen. Besonders vorteilhaft ist es, daß diese wässrige Lösung mit dem ph-Wert > 4,5 unmittelbar vor der Applikation zubereitet wird, um jeglichen Abbau des Wirkstoffes Trospiumchlorid auszuschließen.
Im Gegensatz zu den oben erwähnten anderen Anticholinergika konnte überraschenderweise im Rahmen einer klinischen Studie zum pharmakokinetischen Verhalten von Trospiumchlorid gezeigt werden, daß der Wirkstoff nach intravesikaler Instillation nicht in relevanten Mengen in das Blut übertritt. Selbstverständlich liegt dabei eine ausreichende pharmakologische Wirkung am Blasenmuskel vor. Bei dieser Untersuchung konnten über einen Zeitraum von 12 h nach Instillation von 7,5 mg Trospiumchlorid/20ml zu keinem Meßzeitpunkt Plasmakozentrationen gemessen werden, die oberhalb der Nachweisgrenze der empfindlichen und validierten Meßmethodik lagen. Weiterhin belegen die Ergebnisse einer an Beagle-Hunden durchgeführten Studie zur lokalen Verträglichkeit von Trospiumchlorid nach 1x täglicher intravesikaler Instillation über einen Zeitraum von 4 Wochen, daß die gewählte Rezeptur nicht zu lokalen toxischen Schädigungen des Harnblasengewebes führt. Die Bedeutung einer Zubereitung, die hinsichtlich der gewählten Zusammensetzung, des pH-Wertes und der vorteilhaften Osmolalität der Lösung für eine intravesikale Instillation geeignet ist, verdeutlichen die Ergebnisse weiterer Untersuchungen zur lokalen Verträglichkeit von Trospiumchlorid-Instillationslösung mit abweichender Rezeptur an Beagle-Hunden und von Oxybutynin nach intravesikaler Instillation zerstossener Tabletten an Kanichen (Landau E.H., Fung L.C.T., Thorner P.S., Mittelman M.W., Jayanthi V.R., Churchill B.M., McLorie G.A., Steckler R.E. & Khoury A.E.: Histologic studies of intravesical oxybutynin in the rabbit. J. Urol, 153: 2022-2024, 1995), bei denen bei der histologischen Auswertung Entzündungsprozesse im Bereich des Harnblasengewebes festgestellt wurden.
Im weiteren kommt der Sterilität der in der Blase instillierten Lösung, wie sie bei der Erfindung gegeben ist, im Zusammenhang mit der Vermeidung von Harnwegsinfektionen große Bedeutung zu.

Der Wirkstoff Trospiumchlorid wirkt zum einen als Anticholinergikum, d.h. er besetzt Rezeptoren des parasympathischen Nervensystems, ohne die vom natürlichen Neurotransmitter Acetylcholin induzierten Effekte auszulösen (neurotrope Wirkung). Die anticholinerge Potenz des Trospiumchlorids liegt dabei in vitro um das 20-fache über derjenigen des Oxybutynins (Eckert R.E., Wilhelm A., Schwantes U., Utz J. Alloussi S., Trautwein W. & Ziegler M.: Modulation der zytoplasmatischen Ca2+Konzentration isolierter Myozyten des Detrusor vesicae durch Anticholinergika. Akt. Urol. 26: 4-6, 1995). Zum anderen konnten interne Untersuchungen aufzeigen, daß Trospiumchlorid über eine Inhibition des basalen Ca2+Stromes auch direkt an den glattmuskulären Zellen des Detrusors angreift (muskulotrope Wirkung). Diese muskulotropen Wirkeigenschaften sind gerade bei der direkten Applikation in die Blase von Bedeutung.

## Patentansprüche

1. Verwendung von Trospiumchlorid zur Herstellung einer Arzneizubereitung auf Basis einer sterilen, wässrigen Lösung bei der Behandlung von Blasenfunktionsstörungen und des Harnsystems, gekennzeichnet dadurch, daß das Arzneimittel für die intravesikale Applikation über einen Blasenkatheter direkt in die Blase hinein geeignet ist und aus einer sterilen, wässrigen Lösung des pH-Werts > 4,5 vor der Applikation zubereitet aus einer ersten Komponente von 5,0 - 35,0 mg lyophilisiertem oder gelöstem Trospiumchlorid und einer zweiten Komponente von 20 - 40 ml Natriumchloridlösung mit 0.9 % NaCl besteht.

2. 2-Komponenten-System, umfassend 5,0 - 35 mg Wirkstoff Trospiumchlorid in fester oder gelöster Form als erste Komponente und eine Natriumchloridlösung mit 0,9 % NaCl hierfür als davon getrennte zweite Komponente, in welcher die erste Komponente im Gebrauchszustand auflös- oder verdünnbar und die entstehende Lösung mit einem pH-Wert > 4,5 über einen Blasenkatheter direkt in die Blase instillierbar ist zur Behandlung von Blasenfunktionsstörungen und des Harnsystems.

## Claims

1. Use of trospium chloride for the preparation of a medicinal formulation based on a sterile, aqueous solution in the treatment of bladder disfunctions and the urinary system, characterized in that the medicine is suited for the intravesical administration via a bladder catheter directly into the bladder and contains a sterile, aqueous solution of pH-value > 4.5 and, prior to adminstration, prepared from a first component consisting of 5 to 35 mg of lyophilized or dissolved trospium chloride and a second component consisting of 20 to 40 ml of sodium chloride solution with approximately 0.9 % sodium chloride.

2. 2-component system comprising 5.0 to 35.0 mg of active agent trospium chloride in solid or dissolved form as the first component and a sodium chloride solution with 0.9. % NaCl for the same as a separate, second component, in which the first component can be dissolved or diluted in the use state and the resulting solution, with a ph-value > 4.5, can be introduced directly into the bladder by means of a bladder catheter, for the treatment of bladder disfunctions and the urinary system.

## Revendications

1. Utilisation du chlorure de trospium pour fabriquer une préparation médicamenteuse à base d'une solution aqueuse stérile pour le traitement de troubles de la fonction vésicale et du système urinaire, caractérisé en ce que le médicament convient pour une application intravésicale directement dans la vessie par l'intermédiaire d'une sonde urinaire et en ce qu'il se compose d'une solution aqueuse stérile de pH > 4,5 préparée avant l'application à partir d'un premier composant constitué par 5,0 à 35,0 mg de chlorure de trospium lyophilisé ou dissous et d'un deuxième composant constitué par 20 à 40 ml de solution de chlorure de sodium à 0,9 % de NaCl.

2. Système à deux composants comprenant comme premier composant de 5,0 à 35 mg de principe actif chlorure de trospium sous forme solide ou en solution et, comme deuxième composant séparé du premier, une solution de chlorure de sodium à 0,9 % de NaCl dans laquelle le premier composant dans son état d'utilisation peut être dissous ou dilué et la solution ainsi obtenue, de pH > 4,5, peut être instillée directement dans la vessie par l'intermédiaire d'une sonde urinaire afin de traiter des troubles de la fonction vésicale et du système urinaire.
